# EUROPEAN PATENT SPECIFICATION

(11) **EP 4 373 482 B1**
(45) Date of publication and mention of the grant of the patent: **30.04.2025**
(21) Application number: 22757250.0
(22) Date of filing: 22.07.2022
(51) Int. Cl.: A61K 31/155, A61K 31/522, A61K 9/20, A61P 3/10

(54) **PROCESS FOR PREPARING A PHARMACEUTICAL COMPOSITION COMPRISING LINAGLIPTIN AND METFORMIN HYDROCHLORIDE**
VERFAHREN ZUR HERSTELLUNG EINER PHARMAZEUTISCHEN ZUSAMMENSETZUNG MIT LINAGLIPTIN UND METFORMINHYDROCHLORID
PROCÉDÉ DE PRÉPARATION D'UNE COMPOSITION PHARMACEUTIQUE COMPRENANT DE LA LINAGLIPTINE ET DU CHLORHYDRATE DE METFORMINE

(30) Priority: 22.07.2021 SI 202100144
(43) Date of publication of application: 29.05.2024
(73) Proprietor: KRKA, D.D., Novo Mesto, 8501 Novo mesto (SI)
(72) Inventor: RATEK, Gregor, 8501 NOVO MESTO (SI); VIDMAR, Ana, 8501 NOVO MESTO (SI); SENICA, Luka, 8501 NOVO MESTO (SI); HODNIK, Ziga, 8501 NOVO MESTO (SI); SVETIC, Sandi, 8501 NOVO MESTO (SI); KASTELIC, Aljaz, 8501 NOVO MESTO (SI); KORASA, Klemen, 8501 NOVO MESTO (SI)
(74) Representative: Hoefer & Partner Patentanwälte mbB
(86) International application number: PCT/EP2022/070670
(87) International publication number: WO 2023/002036

(56) References cited:
- WO-A1-2015/110962
- WO-A2-2015/107536
- WO-A2-2019/194773
- US-A1- 2015 283 248

## Description

### FIELD OF THE INVENTION

The present invention relates to a process for preparing a solid pharmaceutical composition comprising linagliptin and metformin hydrochloride.

### BACKGROUND OF THE INVENTION

Linagliptin is a dipeptidyl peptidase-IV (DPP-IV) inhibitor used to treat diabetes mellitus type 2. The structural formula of linagliptin is

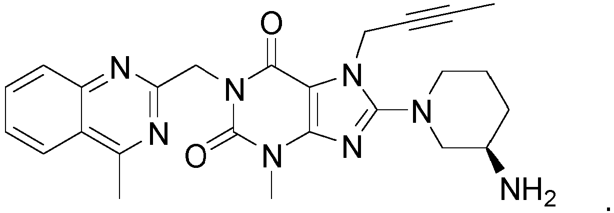

Metformin is a well know compound also used as a medication for the treatment of diabetes mellitus type 2. Metformin is usually administered in its salt form as metformin hydrochloride (HCl). The structural formula of metformin HCl is

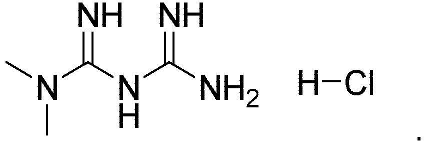

Linagliptin has been first disclosed in the patent application WO2004018468. Its pharmaceutical compositions with selected excipients are disclosed in WO2007128724. Compositions of linagliptin and metformin hydrochloride together with a nucleophilic and/or basic agent for stabilizing linagliptin against degradation are disclosed in WO2009121945. WO2009121945 teaches that linagliptin shows incompatibilities, degradation problems, or extraction problems with a number of excipients which might react with linagliptin amino group. Especially problematic are excipients with reactive carbonyl groups and with carboxylic acid functional groups. Furthermore, WO2009121945 teaches that pre-treatment of metformin HCl by heating/drying reduces an excessive amount of volatile impurities, such as HCl and urea, and therefore improves the stability of linagliptin. Preferred nucleophilic and/or basic agent in the WO2009121945 are basic amino acid, especially L-arginine.

Compositions of linagliptin and metformin HCl which are devoid of basic amino acids are disclosed in WO2015107536 and WO2015110962. In WO2015107536, in one of the examples, a solution of linagliptin, HPMC and propylene glycol in water is prepared and used to coat the seal coated tablets. In WO2015110962, instead of basic amino acids, an alkalizer such as basic magnesium oxide, sodium citrate and meglumine are used to prepare a binder composition comprising linagliptin.

### DETAILED DESCRIPTION OF THE INVENTION

The inventors of the present invention have surprisingly found that linagliptin in formulation comprising linagliptin and metformin HCl, can be stabilized by adding an acid to the granulation liquid comprising linagliptin and spraying thus prepared granulation liquid onto metformin HCl optionally mixed with intragranular excipients to prepare granules.

This is contrary to the teaching of prior art, which teaches that acids are detrimental to the stability of linagliptin and that linagliptin requires stabilization with basic excipients such as basic amino acids, magnesium oxide or meglumine. Furthermore, due to small dose of linagliptin, achieving a suitable content of linagliptin in the final dosage form can pose a problem. In the present invention, an acid is added to the granulation liquid to lower its pH and in that way allow complete dissolution of linagliptin. By preventing the loss of linagliptin due to sedimentation of undissolved linagliptin in granulation liquid and its deposits in different parts of granulation equipment, this procedure beneficially contributes to the provision of a desired content of linagliptin, and if desired, beneficially contributes to the provision of even high contents of linagliptin, in the final dosage form.

The object of the present invention is a process for preparing a solid pharmaceutical composition comprising linagliptin and metformin hydrochloride, wherein the process comprises the following steps:
a) preparing a granulation liquid by dispersing linagliptin, an acid and optionally a binder in water;
b) spraying the granulation liquid onto metformin hydrochloride optionally mixed with intragranular excipient(s) to prepare granules;
c) drying the granules;
d) optionally adding extragranular excipient(s); and
e) preparing the final dosage form.

The granulation liquid comprises linagliptin, water, an acid and optionally a binder. Preferably, the granulation liquid comprises also a binder. The binder is dispersed in the granulation liquid.

In an advantageous embodiment, the linagliptin is completely dissolved in the granulation liquid prepared in step a). In a further embodiment, both the linagliptin and the binder are completely dissolved in the granulation liquid prepared in step a).

In particular, step a) can be: a) preparing a granulation liquid by dispersing linagliptin, an acid, and a binder in water.

Especially, step b) can be or can comprise: b) spraying the granulation liquid onto metformin hydrochloride (preferably in the form of solid particles) or onto metformin hydrochloride mixed with intragranular excipient(s) (preferably the mixture of metformin hydrochloride and intragranular excipient(s) is in the form of a mixture of solid particles or in the form of a fluid comprising solid particles) to prepare granules.

The spraying procedure of step b) can be carried out in such a manner that during step b) at least one of metformin hydrochloride and the one or more optionally present intragranular excipient(s) is not completely dissolved.

The final dosage form prepared in step e) can be any dosage form comprising the granules comprising linagliptin and metformin HCl (in particular obtained in step c)) and optionally the extragranular excipient(s) optionally added in step d).

For example, the final dosage form can be e.g. one of
- granules comprising linagliptin and metformin HCl (in particular obtained in step c)),
- a capsule filled with said granules or a tablet obtained by compressing said granules,
- a mixture of granules comprising linagliptin and metformin HCl (in particular obtained in step c)) and the extragranular excipient(s) (in particular as added in step d)),
- a capsule filled with a mixture of granules comprising linagliptin and metformin HCl (in particular obtained in step c)) and the extragranular excipient(s) (in particular as added in step d)) or a tablet obtained by compressing said mixture.

In particular, step e) can be or comprise: e) preparing the final dosage form by mixing the granules obtained in step c) and the extragranular excipient(s) added in step d).

The so obtained mixture comprising the granules and the extragranular excipient(s) can be filled into a capsule or compressed to obtain a tablet.

In an embodiment, the final dosage form is a tablet, and step e) can be or comprise:
e) preparing the final dosage form by
i) mixing the granules (obtained in step c)) and the extragranular excipient(s) (added in step d)), and subjecting the so obtained mixture to compression to prepare a tablet, or
ii) subjecting the granules (obtained in step c)) to compression to prepare a tablet.

The tablet can be optionally provided with a coating.

The term "acid" as used herein refers to a compound that can donate a proton (in particular can donate a proton to H₂O) and has a pKa of less than 6. pKa being -log₁₀ Ka, as may be seen from the following equation: pKa = -log₁₀ Ka. (Ka being also known as "acid dissociation constant".) pKa values can be found in standard tables, for example in CRC Handbook of Chemistry and Physics, W.M.Haynes, 97th ed., sections 5-87 and 5-88. The pKa can be determined at 25°C by titration, spectrophotometric and/or NMR method. In an embodiment, the experimental determination of pKa values can be performed by titration at constant temperature (in particular 25°C), in particular in a medium of high ionic strength. Reference can be for example made to Wikipedia, "Acid dissociation constant", version of 12 July 2021). In particular, a procedure can be or comprise: A solution of the compound (for which the pKa value is to be determined) in a medium is acidified with a strong acid until the compound is fully protonated. The solution can be then titrated with a strong base until all the protons have been removed. At several (e.g. 20) points in time during titration, pH can be measured (using e.g. a glass electrode and a pH meter). Subsequently, the equilibrium constants can be e.g. obtained by fitting calculated pH values to the observed values, in particular using the method of least squares (Leggett, D.J. (1985) Computational Methods for the Determination of Formation Constants, Plenum, ISBN 0-306-41957-2) The total volume of added strong base should be small compared to the initial volume of titrand solution for keeping the ionic strength constant or essentially constant. If a glass electrode can not be employed, spectrophotometric methods can be used (Reference is made e.g. to Kütt, A.; Leito, I.; Kaljurand, I.; et al. (2006), "A Comprehensive Self-Consistent Spectrophotometric Acidity Scale of Neutral Brønsted Acids in Acetonitrile", J. Org. Chem. 71 (7): 2829-2838) Spectrophotometric methods can comprise absorbance or fluorescence measurements. In case of absorbance measurements, the Beer-Lambert law is assumed to be applicable.

In one embodiment, the acid can be an acid which has a pKa which is less than the pKa of hexanoic acid (the pKa for said acids can be determined under the same measurement conditions at a temperature of 25°C). The acid can be organic or inorganic acid. Preferably, the acid is selected from the group consisting of hydrochloric acid, citric acid, fumaric acid, maleic acid, malic acid, succinic acid, phosphoric acid and/or acetic acid. More preferably, the acid is hydrochloric acid and/or citric acid. Most preferably, the acid is hydrochloric acid (HCl). The acid can be added to the granulation liquid in its pure form or as an aqueous solution. Preferably, the acid is added to the granulation liquid as an aqueous solution.

To form the granulation liquid, linagliptin may first be suspended in water and then acid is added to dissolve linagliptin. Preferably, linagliptin is added to the acidic aqueous solution which speeds up its dissolution. The acidic aqueous solution comprises water and an acid.

The pH of the granulation liquid can be in the range of 3 to 8, preferably 4 to 7, and most preferably in the range of 5 to 7, especially in the range 5.0 to 7.0. The pH is measured at room temperature (about 25 °C, in particular 25°C) by a Mettler Toledo SevenCompact pH meter S220 using a Mettler Toledo general glass pH electrode InLab Routine. The pH of the granulation liquid can be adjusted by adding more acid to the granulation liquid.

Pharmaceutical composition according to the present invention can comprise excipients, which can be binders, diluents, disintegrants, glidants and/or lubricants.

The optionally present intragranular excipient(s) and the optionally present extragranular excipient(s) can be free of arginine, lysine, histidine, and magnesium oxide, optionally free of basic amino acids and magnesium oxide, further optionally free of amino acids and magnesium oxide. Additionally or alternatively, the optionally present intragranular excipient(s) and the optionally present extragranular excipient(s) can be free of amino sugars. The granulation liquid used in step b) can be free of arginine, lysine, histidine, and magnesium oxide, optionally free of basic amino acids and magnesium oxide, further optionally free of amino acids and magnesium oxide. Additionally or alternatively, the granulation liquid used in step b) can be free of amino sugars.

The pharmaceutical composition according to the present invention can be free of arginine, lysine, histidine, optionally free of basic amino acids, further optionally free of amino acids. Additionally or alternatively, the pharmaceutical composition according to the present invention can be free of amino sugars. The granules comprising linagliptin and metformin HCl (in particular as obtained in step c)) can be free of arginine, lysine, histidine, optionally free of basic amino acids, further optionally free of amino acids. Additionally or alternatively, the granules comprising linagliptin and metformin HCl (in particular as obtained in step c)) can be free of amino sugars.

Binder(s), in particular binder(s) used for preparing the granulation liquid can be or comprise binder(s) selected from the group consisting of povidone (polyvinylpyrrolidone), copovidone, hydroxypropylmethyl cellulose (HPMC), hydroxypropyl cellulose (HPC), hydroxyethyl cellulose, methyl cellulose, polyvinyl alcohol, polyethylene glycol, starch and/or pregelatinized starch. Preferably, the binder (in particular the binder used for preparing the granulation liquid) is copovidone and/or povidone, most preferably the binder is copovidone. The amount of binder in the pharmaceutical composition according to the present invention can be within the range of from 4 to 20 % by weight, preferably from 5 to 15 % by weight and most preferably from 6 to 14 % by weight of the final dosage form.

Binder(s) used as intragranular excipient(s) and/or as extragranular excipient(s) can be or comprise binder(s) selected from the group consisting of povidone (polyvinylpyrrolidone), copovidone, hydroxypropylmethyl cellulose (HPMC), hydroxypropyl cellulose (HPC), hydroxyethyl cellulose, methyl cellulose, polyvinyl alcohol, polyethylene glycol, starch and/or pregelatinized starch. Preferably, the binder is copovidone and/or povidone, most preferably the binder is copovidone.

Optionally, the intragranular excipient(s) can comprise diluent(s). Optionally, the extragranular excipient(s) can comprise diluent(s). Diluent(s), especially one or both of diluent(s) used as extragranular excipient(s) and diluent(s) used as intragranular excipient(s), can be selected from starch and its derivatives (e.g., corn starch, pregelatinized starch), cellulose, microcrystalline cellulose, co-processed microcrystalline cellulose such as silicified microcrystalline cellulose, carbohydrates or their derivatives such as mannitol, xylitol, isomalt, sorbitol, lactose, dextrose, dextrates, dextrin, sucrose, metal salts of phosphoric adic (e.g., calcium hydrogenphosphate in anhydrous or hydrated form) or other diluents and combinations thereof (which can be specifically listed herein or not specifically listed herein). Preferably, the diluent, especially the diluent which is one of the extragranular excipient(s) and/or one of the intragranular excipient(s), is microcrystalline cellulose.

Optionally, the intragranular excipient(s) can comprise disintegrant(s). Optionally, the extragranular excipient(s) can comprise disintegrant(s). Disintegrant(s), especially one or both of disintegrant(s) used as extragranular excipient(s) and disintegrant(s) used as intragranular excipient(s), can be selected from the group consisting of crospovidone, pregelatinized starch, croscarmellose sodium, carmellose sodium or calcium, low substituted hydroxypropyl cellulose, sodium starch glycolate, salts of polacrilin such as potassium polacrilin, and silicate derivatives such as calcium silicate or other disintegrants or combinations thereof (which can be specifically listed herein or not specifically listed herein). Preferably, the disintegrant, especially the disintegrant which is one of the extragranular excipient(s) and/or one of the intragranular excipient(s), is croscarmellose sodium.

Optionally, the intragranular excipient(s) can comprise lubricant(s). Optionally, the extragranular excipient(s) can comprise lubricant(s). Lubricant(s), especially one or both of lubricant(s) used as extragranular excipient(s) and lubricant(s) used as intragranular excipient(s), can be selected from the group consisting of stearic acid, a metal salt of stearic acid such as magnesium stearate, talc, colloidal silicon dioxide, a wax variety such as beads wax, spermaceti, boric acid, adipic acid, sodium sulphate, fumaric acid, stearyl sodium fumarate, sucrose aliphatic acid ester, a lauryl sulphate such as sodium lauryl sulphate, magnesium lauryl sulphate, starches or starch derivatives (such as corn starch, potato starch), glyceryl behenate, behenoyl polyoxyl glyceride etc., and combinations thereof. Preferred lubricants are metal salt of stearic acid such as magnesium stearate, stearic acid, sodium stearyl fumarate or their mixture, preferably magnesium stearate. Lubricants can be present in an amount from about 0.1% by weight to 5% by weight of the composition, preferably from 0.25% by weight to 3% by weight.

Optionally, the intragranular excipient(s) can comprise glidant(s). Optionally, the extragranular excipient(s) can comprise glidant(s). Glidant(s), especially one or both of glidant(s) used as extragranular excipient(s) and glidant(s) used as intragranular excipient(s), can be selected from the group consisting of silicon dioxide, talc, magnesium trisilicate, magnesium silicate, calcium phosphate, powdered cellulose, starch, talc, and/or magnesium oxide. Preferably, the glidant, especially e.g. the glidant used as extragranular excipient or e.g. the glidant used as intragranular excipient, is silicon dioxide and most preferably, the glidant is colloidal silicon dioxide.

In step b) of the process according to the present invention, the granulation liquid can be sprayed onto metformin hydrochloride mixed with intragranular excipient(s) to prepare the granules. The intragranular excipient(s) can be selected from excipients as disclosed herein above, e.g. can be one or more intragranular excipients selected from binder(s), diluent(s), disintegrant(s), lubricant(s), and glidant(s). Preferably, the one or more intragranular excipient(s) are selected from starch, mannitol, microcrystalline cellulose, croscarmellose sodium and/or colloidal silicon dioxide. Especially, the intragranular excipient(s) can be or can comprise one or more diluents. Most preferably, the intragranular excipient is starch.

Alternatively, the granulation liquid in step b) of the process according to the present invention can be sprayed onto metformin hydrochloride only without additional intragranular excipients to prepare the granules.

In step d) of the process according to the present invention, extragranular excipients can be added. The extragranular excipients can be selected from excipients as disclosed herein above. For example, the extragranular excipient(s) can comprise one or more excipients selected from the group consisting of binder(s), diluent(s), disintegrant(s), glidant(s), and lubricant(s). The extragranular excipient(s) can comprise one or more extragranular excipient(s) which are selected from mannitol, microcrystalline cellulose, isomalt, povidone, croscarmellose sodium, colloidal silicon dioxide and/or magnesium stearate. Preferably, the extragranular excipient(s) are selected from mannitol, microcrystalline cellulose, isomalt, povidone, croscarmellose sodium, colloidal silicon dioxide and/or magnesium stearate. Most preferably, the extragranular excipient(s) are microcrystalline cellulose, colloidal silicon dioxide and/or magnesium stearate. Furthermore, the extragranular excipient(s) can comprise at least two or all of microcrystalline cellulose, colloidal silicon dioxide and magnesium stearate.

Most preferable aspect of the present invention is a process, wherein the pH of the granulation liquid is in the range of 5 to 7, the acid is hydrochloric acid, the binder is copovidone and extragranular excipient is magnesium stearate.

The final dosage form can be a capsule or a tablet. Preferably, the final dosage form is a tablet. The tablet can be prepared by compression of the mixture obtained after step d) of the process according to the present invention. In particular, the tablet can be e.g. prepared by mixing the granules obtained in step c) and the extragranular excipient(s) added in step d), and subjecting the so obtained mixture to compression to obtain a tablet. The tablet can be film coated with conventional materials used for film coating. Film coating can be used for achieving smoother and/or colored tablet surfaces resulting in an easier swallowing of the tablets and or distinguishing among different strengths or pharmaceutical products. Polymers which can be used for film coating can be selected from hydroxypropylmethyl cellulose, hydroxypropyl cellulose, polyvinyl alcohol, copovidone and/or povidone. For example, commercially available film coating sold under the trade name Opadry^{®} can be used (as an aqueous or ethanolic dispersion) to coat the tablet.

The amount of linagliptin in the pharmaceutical composition according to the present invention can be within the range of from 0.1 to 0.5 % by weight, preferably from 0.1 to 0.4 % by weight and most preferably from 0.1 to 0.3 % by weight of the final dosage form. Alternatively, the amount of linagliptin in the pharmaceutical composition according to the present invention can be in the range of 0.5 mg to 10 mg, preferably 0.5 mg, 1 mg, 2.5 mg, 5 mg or 10 mg. Most preferably, the amount of linagliptin is 2.5 mg.

The amount of metformin hydrochloride in the pharmaceutical composition according to the present invention can be in the range of 250 mg to 1000 mg, preferably 250, 500, 625, 750, 850 or 1000 mg. Most preferably, the amount of metformin hydrochloride is in the range of from 850 to 1000 mg, especially is 850 or 1000 mg.

The granules prepared in step c) comprise linagliptin and metformin hydrochloride, the weight ratio linagliptin to metformin hydrochloride (linagliptin : metformin hydrochloride) can be in the range 1:2000 to 1:25.

The final dosage form (in particular tablet or capsule) prepared in step e) can comprise linagliptin in an amount in the range of from 0.5 mg to 10 mg, and metformin hydrochloride in an amount in the range of from 250 to 1000 mg.

According to a further aspect, the present inventors provide a solid pharmaceutical composition comprising linagliptin and metformin hydrochloride, which is obtainable by a process as disclosed herein, especially obtainable by a process comprising the following steps:
a) preparing a granulation liquid by dispersing linagliptin, an acid and optionally a binder in water;
b) spraying the granulation liquid onto metformin hydrochloride optionally mixed with intragranular excipient(s) to prepare granules;
c) drying the granules;
d) optionally adding extragranular excipient(s); and
e) preparing the final dosage form.

According to a still further aspect, the present inventors provide this solid pharmaceutical composition comprising linagliptin and metformin hydrochloride (which is obtainable by a process as disclosed herein, especially obtainable by a process comprising steps a) to e) as defined herein supra) for use in the treatment of diabetes mellitus type 2.

In particular, there are provided the following items:
1. A process for preparing a solid pharmaceutical composition comprising linagliptin and metformin hydrochloride, wherein the process comprises the following steps:
   a) preparing a granulation liquid by dispersing linagliptin, an acid and optionally a binder in water;
   b) spraying the granulation liquid onto metformin hydrochloride optionally mixed with intragranular excipient(s) to prepare granules;
   c) drying the granules;
   d) optionally adding extragranular excipient(s); and
   e) preparing the final dosage form.
2. The process according to item 1, wherein the granulation liquid comprises also a binder.
3. The process according to item 2, wherein the binder is or comprises binder selected from the group consisting of povidone, copovidone, hydroxypropylmethyl cellulose, hydroxypropyl cellulose, hydroxyethyl cellulose, methyl cellulose, polyvinyl alcohol, polyethylene glycol, starch and/or pregelatinized starch.
4. The process according to item 3, wherein the binder is copovidone.
5. The process according to any one of the previous items, wherein the pH of the granulation liquid is in the range of 3 to 8.
6. The process according to item 5, wherein the pH of the granulation liquid is in the range of 4 to 7.
7. The process according to item 6, wherein the pH of the granulation liquid is in the range of 5 to 7.
8. The process according to any one of the previous items, wherein the acid is organic or inorganic acid.
9. The process according to item 8, wherein the acid is selected from the group consisting of hydrochloric acid, citric acid, fumaric acid, maleic acid, malic acid, succinic acid, phosphoric acid and/or acetic acid.
10. The process according to item 9, wherein the acid is hydrochloric acid and/or citric acid.
11. The process according to item 10, wherein the acid is hydrochloric acid.
12. The process according to any one of the previous items, wherein the granulation liquid in step b) is sprayed onto metformin hydrochloride only without additional intragranular excipients to prepare the granules.
13. The process according to any one of the previous items, wherein the extragranular excipients are magnesium stearate and/or colloidal silicon dioxide.
14. The process according to any one of the previous items, wherein the pH of the granulation liquid is in the range of 5 to 7, the acid is hydrochloric acid, the binder is copovidone and extragranular excipient is magnesium stearate.
15. The process according to any one of the previous items, wherein the final dosage form is a tablet.
16. Solid pharmaceutical composition comprising linagliptin and metformin hydrochloride, which is obtainable by a process according to any one of the preceding items 1 to 15.
17. Solid pharmaceutical composition comprising linagliptin and metformin hydrochloride, which is obtainable by a process according to any one of items 1 to 15, for use in the treatment of diabetes mellitus type 2.
18. Solid pharmaceutical composition comprising linagliptin and metformin hydrochloride, wherein the solid pharmaceutical composition further comprises an acid and optionally further comprises a binder.
19. Solid pharmaceutical composition according to item 18, said solid pharmaceutical composition being a solid pharmaceutical composition comprising granules and extragranular excipient(s),
   said granules comprise linagliptin, metformin hydrochloride, and further comprise an acid, one or more binders and optionally one or more diluents, and
   said extragranular excipient(s) optionally comprise one or more diluent(s), one or more lubricant(s) and one or more glidant(s).
20. Solid pharmaceutical composition according to item 18 or 19, wherein the granules comprise one or more diluents.
21. Solid pharmaceutical composition according to any of items 18 to 20, wherein the granules comprise one or more diluents, and said one or more diluents being or comprising microcrystalline cellulose.
22. Solid pharmaceutical composition according to any of items 18 to 21 which is obtainable by any one of the processes of items 1 to 15.

The invention is illustrated by the following non-limiting examples:

### EXAMPLES

### Comparative example 1: Film coated tablets comprising linagliptin and metformin hydrochloride prepared without an acid in the granulation liquid

| Ingredients | mg / dry tablet |
|---|---|
| Linagliptin | 2.50 |
| Copovidone | 95.00 |
| Metformin hydrochloride | 1000.00 |
| Starch | 42.50 |
| Colloidal silicon dioxide | 5.00 |
| Magnesium stearate | 10.00 |
| Opadry^{®} | 19.95 |
| Iron oxide, red | 0.05 |

Solution of copovidone in purified water was prepared. Linagliptin was added and mixed until homogeneous suspension with a pH of around 9 was obtained. Thus prepared granulation liquid was sprayed onto a mixture of metformin hydrochloride and starch by top spraying process to obtain granules, which were dried and sieved through 450 mesh sieve to obtain uniform dry granules. The granules were blended with colloidal silicon dioxide to obtain a mixture, which was further lubricated with magnesium stearate to obtain compression mixture, which was compressed into tablets. The tablets were coated with Opadry and red iron oxide as an aqueous dispersion by conventional coating process.

### Example 1: Film coated tablets comprising linagliptin and metformin hydrochloride

| Ingredients | mg / dry tablet |
|---|---|
| Linagliptin | 2.50 |
| Copovidone | 95.00 |
| Metformin hydrochloride | 1000.00 |
| Starch | 42.50 |
| Colloidal silicon dioxide | 5.00 |
| Magnesium stearate | 10.00 |
| Opadry^{®} | 19.95 |
| Iron oxide, red | 0.05 |

Purified water was slightly acidified with 0.5 M HCl_{(aq)} to a pH of 6. Linagliptin was added and mixed until completely dissolved. Copovidone was added and mixed until completely dissolved. The pH was adjusted to 7 with 0.5 M HCl_{(aq)}. Thus prepared granulation liquid was sprayed onto a mixture of metformin hydrochloride and starch by top spraying process to obtain granules, which were dried and sieved through 450 mesh sieve to obtain uniform dry granules. The granules were blended with colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

### Example 2: Film coated tablets comprising linagliptin and metformin hydrochloride

| Ingredients | mg / dry tablet |
|---|---|
| Linagliptin | 2.50 |
| Copovidone | 95.00 |
| Metformin hydrochloride | 1000.00 |
| Starch | 42.50 |
| Colloidal silicon dioxide | 5.00 |
| Magnesium stearate | 10.00 |
| Opadry^{®} | 19.95 |
| Iron oxide, red | 0.05 |

Purified water was slightly acidified with 0.5 M HCl_{(aq)} to a pH of 6. Linagliptin was added and mixed until completely dissolved. Copovidone was added and mixed until completely dissolved. The pH was adjusted to 6 with 0.5 M HCl_{(aq)}. Thus prepared granulation liquid was sprayed onto a mixture of metformin hydrochloride and starch by top spraying process to obtain granules, which were dried and sieved through 450 mesh sieve to obtain uniform dry granules. The granules were blended with colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

### Stability testing

In the table below, results (sum of HPLC peak areas for impurities of linagliptin) of the stability testing of the blister packed tablets obtained according to Comparative example 1, Example 1 and Example 2 under accelerated conditions are shown:

| Stability Testing Conditions | Comp. ex. 1 | Ex. 1 | Ex. 2 |
|---|---|---|---|
| Initial | < 0.10% | < 0.10% | < 0.10% |
| 1 month at 50°C/75% RH, Alu/Alu blisters | 1.36% | 0.44% | 0.25% |
| 3 months at 40°C/75% RH, Alu/Alu blisters | 1.55% | 0.40% | 0.34% |

The analytical method used for determination of linagliptin impurities was a gradient HPLC method, using YMC-Triart Bio column (250 mm × 4.6 mm; 3 µm) with stationary phase C18 and UV detection at wavelength 296 nm. The mobile phase was a combination of acetonitrile and 0.01 M ammonium format buffer with pH 3.0.

As is evident from the results of stability testing, tablets of Examples 1 and 2 are more stable than tablets of Comparative example 1.

### Comparative example 2: Film coated tablets comprising linagliptin and metformin hydrochloride prepared without an acid in the granulation liquid

| Ingredients | mg / dry tablet |
|---|---|
| Linagliptin | 2.50 |
| Copovidone | 95.00 |
| Metformin hydrochloride | 1000.00 |
| Cellulose, microcrystalline | 92.50 |
| Croscarmellose sodium | 25.00 |
| Colloidal silicon dioxide | 5.00 |
| Magnesium stearate | 10.00 |
| Opadry^{®} | 19.95 |
| Iron oxide, red | 0.05 |

Solution of copovidone in purified water was prepared. Linagliptin was added and mixed until homogeneous suspension with a pH around 9 was obtained. Thus prepared granulation liquid was sprayed onto metformin hydrochloride by top spraying process to obtain granules, which were dried and sieved through 600 mesh sieve to obtain uniform dry granules. The granules were blended with microcrystalline cellulose, croscarmellose sodium and colloidal silicon dioxide to obtain a mixture, which was further lubricated with magnesium stearate to obtain compression mixture, which was compressed into tablets. The tablets were coated with Opadry and red iron oxide as an aqueous dispersion by conventional coating process.

### Example 3: Film coated tablets comprising linagliptin and metformin hydrochloride

| Ingredients | mg / dry tablet |
|---|---|
| Linagliptin | 2.50 |
| Copovidone | 95.00 |
| Metformin hydrochloride | 1000.00 |
| Cellulose, microcrystalline | 92.50 |
| Croscarmellose sodium | 25.00 |
| Colloidal silicon dioxide | 5.00 |
| Magnesium stearate | 10.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Copovidone was added and mixed until completely dissolved. The pH was adjusted to 5 with 0.5 M HCl_{(aq)}. Thus prepared granulation liquid was sprayed onto metformin hydrochloride by top spraying process to obtain granules, which were dried and sieved through 600 mesh sieve to obtain uniform dry granules. The granules were blended with microcrystalline cellulose, croscarmellose sodium and colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

### Assay results

In the table below, assay results of Comparative example 2 and Example 3 are presented:

| Test | Comp. ex. 2 | Ex. 3 |
|---|---|---|
| Content of metformin hydrochloride | 99.7% | 99.3% |
| Content of linagliptin | 91.5% | 99.2% |

The analytical method used for determination of linagliptin and metformin hydrochloride content was a gradient HPLC method, using X-Bridge column (150 mm x 4.6 mm; 3.5 µm) with stationary phase C18 and UV detection at wavelength 226 nm. The mobile phase was a combination of acetonitrile and sodium phosphate buffer with pH 2.0 containing octanesulfonic acid sodium salt and sodium dihydrogen phosphate

As it is evident from the assay results, tablets of Example 3 gave better results regarding content of linagliptin in comparison to tablets of Comparative example 2.

### Stability testing

In the table below, results (sum of HPLC peak areas for impurities of linagliptin) of the stability testing of the blister packed tablets obtained according to Comparative example 2 and Example 3 under accelerated conditions are shown:

| Stability Testing Conditions | Comp. ex. 2 | Ex. 3 |
|---|---|---|
| 6 months at 40°C/75% RH, PVC/PVDC blisters | 0.83 % | 0.39 % |

The analytical method used for determination of linagliptin impurities was the same as for Comp. ex. 1, Ex. 1 and Ex. 2.

As is evident from the results of stability testing, tablets of Example 3 are more stable than tablets of Comparative example 2.

### Example 4: Film coated tablets comprising linagliptin and metformin hydrochloride

| Ingredients | mg / dry tablet |
|---|---|
| Linagliptin | 2.50 |
| Copovidone | 95.00 |
| Metformin hydrochloride | 1000.00 |
| Cellulose, microcrystalline | 92.50 |
| Croscarmellose sodium | 25.00 |
| Colloidal silicon dioxide | 5.00 |
| Magnesium stearate | 10.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Copovidone was added and mixed until completely dissolved. The pH was adjusted to 5 with 0.5 M HCl_{(aq)}. Thus prepared granulation liquid was sprayed onto metformin hydrochloride by top spraying process to obtain granules, which were dried and sieved through 600 mesh sieve to obtain uniform dry granules. The granules were blended with microcrystalline cellulose, croscarmellose sodium and colloidal silicon dioxide to obtain a mixture which was further lubricated with magnesium stearate to obtain compression mixture which was compressed into tablets. The tablets were coated with Opadry and red iron oxide as an ethanolic/aqueous dispersion by conventional coating process.

### Example 5: Film coated tablets comprising linagliptin and metformin hydrochloride

| Ingredients | mg / dry tablet |
|---|---|
| Linagliptin | 2.50 |
| Copovidone | 82.00 |
| Metformin hydrochloride | 850.00 |
| Cellulose, microcrystalline | 81.50 |
| Croscarmellose sodium | 21.25 |
| Colloidal silicon dioxide | 4.25 |
| Magnesium stearate | 8.50 |
| Opadry^{®} | 16.95 |
| Iron oxide, yellow | 0.05 |

Purified water was acidified with 0.5 M HCl_{(aq)} to a pH of 2.5. Linagliptin was added and mixed. Dispersion was further acidified until transparent solution was obtained. Copovidone was added and mixed until completely dissolved. The pH was adjusted to 5 with 0.5 M HCl_{(aq)}. Thus prepared granulation liquid was sprayed onto metformin hydrochloride by top spraying process to obtain granules, which were dried and sieved through 600 mesh sieve to obtain uniform dry granules. The granules were blended with microcrystalline cellulose, croscarmellose sodium and colloidal silicon dioxide to obtain a mixture which was further lubricated with magnesium stearate to obtain compression mixture which was compressed into tablets. The tablets were coated with Opadry and yellow iron oxide as an aqueous dispersion by conventional coating process.

### Example 6: Film coated tablets comprising linagliptin and metformin hydrochloride

| Ingredients | mg / dry tablet |
|---|---|
| Linagliptin | 2.50 |
| Copovidone | 85.00 |
| Metformin hydrochloride | 1000.00 |
| Cellulose, microcrystalline | 42.50 |
| Mannitol | 50.00 |
| Colloidal silicon dioxide | 5.00 |
| Magnesium stearate | 15.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Copovidone was added and mixed until completely dissolved. The pH was adjusted to 6 with 0.5 M HCl_{(aq)}. Thus prepared granulation liquid was sprayed onto a mixture of metformin hydrochloride and microcrystalline cellulose by top spraying process to obtain granules, which were dried and sieved through 600 mesh sieve to obtain uniform dry granules. The granules were blended with mannitol and colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

### Example 7: Film coated tablets comprising linagliptin and metformin hydrochloride

| Ingredients | mg / dry tablet |
|---|---|
| Linagliptin | 2.50 |
| Copovidone | 85.00 |
| Citric acid | 1.50 |
| Metformin hydrochloride | 1000.00 |
| Cellulose, microcrystalline | 42.50 |
| Mannitol | 50.00 |
| Colloidal silicon dioxide | 5.00 |
| Magnesium stearate | 15.00 |
| Opadry^{®} | 19.95 |
| Iron oxide, red | 0.05 |

Purified water was acidified with citric acid to a pH of 6. Linagliptin was added and mixed. Dispersion was further acidified until transparent solution was obtained. Copovidone was added and mixed until completely dissolved. The pH was adjusted to 6 with citric acid. Thus prepared granulation liquid was sprayed onto a mixture of metformin hydrochloride and microcrystalline cellulose by top spraying process to obtain granules, which were dried and sieved through 600 mesh sieve to obtain uniform dry granules. The granules were blended with mannitol and colloidal silicon dioxide to obtain a mixture which was further lubricated with magnesium stearate to obtain compression mixture which was compressed into tablets. The tablets were coated with Opadry and red iron oxide as an ethanolic/aqueous dispersion by conventional coating process.

### Example 8: Film coated tablets comprising linagliptin and metformin hydrochloride

| Ingredients | mg / dry tablet |
|---|---|
| Linagliptin | 2.50 |
| Povidone | 85.00 |
| Metformin hydrochloride | 1000.00 |
| Cellulose, microcrystalline | 42.50 |
| Mannitol | 50.00 |
| Colloidal silicon dioxide | 5.00 |
| Magnesium stearate | 15.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Povidone was added and mixed until completely dissolved. The pH was adjusted to 6 with 0.5 M HCl_{(aq)}. Thus prepared granulation liquid was sprayed onto a mixture of metformin hydrochloride and microcrystalline cellulose by top spraying process to obtain granules, which were dried and sieved through 600 mesh sieve to obtain uniform dry granules. The granules were blended with mannitol and colloidal silicon dioxide to obtain a mixture which was further lubricated with magnesium stearate to obtain compression mixture which was compressed into tablets. The tablets were coated with Opadry and red iron oxide as an ethanolic/aqueous dispersion by conventional coating process.

### Example 9: Film coated tablets comprising linagliptin and metformin hydrochloride

| Ingredients | mg / dry tablet |
|---|---|
| Linagliptin | 2.50 |
| Copovidone | 110.50 |
| Metformin hydrochloride | 850.00 |
| Cellulose, microcrystalline | 85.90 |
| Croscarmellose sodium | 43.35 |
| Colloidal silicon dioxide | 4.25 |
| Magnesium stearate | 8.50 |
| Opadry^{®} | 21.70 |
| Iron oxide, red | 0.10 |
| Iron oxide, yellow | 0.20 |

Purified water was acidified with 0.5 M HCl_{(aq)} to a pH of 2.5. Linagliptin was added and mixed. Dispersion was further acidified until transparent solution was obtained. Copovidone was added and mixed until completely dissolved. The pH was adjusted to 4 with 0.5 M HCl_{(aq)}. Thus prepared granulation liquid was sprayed onto metformin hydrochloride by top spraying process to obtain granules, which were dried and sieved through 600 mesh sieve to obtain uniform dry granules. The granules were blended with microcrystalline cellulose, croscarmellose sodium and colloidal silicon dioxide to obtain a mixture which was further lubricated with magnesium stearate to obtain compression mixture which was compressed into tablets. The tablets were coated with Opadry, red and yellow iron oxide as an aqueous dispersion by conventional coating process.

### Example 10: Film coated tablets comprising linagliptin and metformin hydrochloride

| Ingredients | mg / dry tablet |
|---|---|
| Linagliptin | 2.50 |
| Hydroxypropyl cellulose | 80.00 |
| Metformin hydrochloride | 1000.00 |
| Cellulose, microcrystalline | 42.50 |
| Mannitol | 55.00 |
| Colloidal silicon dioxide | 5.00 |
| Magnesium stearate | 15.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Hydroxypropyl cellulose (HPC) was added and mixed until completely dissolved. The pH was adjusted to 6 with 0.5 M HCl_{(aq)}. Thus prepared granulation liquid was sprayed onto a mixture of metformin hydrochloride and microcrystalline cellulose by top spraying process to obtain granules, which were dried and sieved through 600 mesh sieve to obtain uniform dry granules. The granules were blended with mannitol and colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

### Example 11: Film coated tablets comprising linagliptin and metformin hydrochloride

| Ingredients | mg / dry tablet |
|---|---|
| Linagliptin | 2.50 |
| Hydroxypropyl cellulose | 80.00 |
| Metformin hydrochloride | 1000.00 |
| Cellulose, microcrystalline | 42.50 |
| Mannitol | 55.00 |
| Sodium starch glycolate | 25.00 |
| Colloidal silicon dioxide | 5.00 |
| Magnesium stearate | 18.00 |
| Opadry^{®} | 19.95 |
| Iron oxide, red | 0.05 |

Purified water was acidified with 0.5 M HCl_{(aq)} to a pH of 2.5. Linagliptin was added and mixed. Dispersion was further acidified until transparent solution was obtained. Hydroxypropyl cellulose (HPC) was added and mixed until completely dissolved. The pH was adjusted to 6 with 0.5 M HCl_{(aq)}. Thus prepared granulation liquid was sprayed onto a mixture of metformin hydrochloride and microcrystalline cellulose by top spraying process to obtain granules, which were dried and sieved through 600 mesh sieve to obtain uniform dry granules. The granules were blended with mannitol, sodium starch glycolate and colloidal silicon dioxide to obtain a mixture which was further lubricated with magnesium stearate to obtain compression mixture which was compressed into tablets. The tablets were coated with Opadry and red iron oxide as an aqueous dispersion by conventional coating process.

## Claims

1. A process for preparing a solid pharmaceutical composition comprising linagliptin and metformin hydrochloride, wherein the process comprises the following steps:
a) preparing a granulation liquid by dispersing linagliptin, an acid and optionally a binder in water;
b) spraying the granulation liquid onto metformin hydrochloride optionally mixed with intragranular excipient(s) to prepare granules;
c) drying the granules;
d) optionally adding extragranular excipient(s); and
e) preparing the final dosage form.

2. The process according to claim 1, wherein the granulation liquid comprises also a binder.

3. The process according to claim 2, wherein the binder is or comprises binder selected from the group consisting of povidone, copovidone, hydroxypropylmethyl cellulose, hydroxypropyl cellulose, hydroxyethyl cellulose, methyl cellulose, polyvinyl alcohol, polyethylene glycol, starch and/or pregelatinized starch.

4. The process according to claim 3, wherein the binder is copovidone.

5. The process according to any one of the previous claims, wherein the pH of the granulation liquid is in the range of 3 to 8, optionally, wherein the pH of the granulation liquid is in the range of 4 to 7, further optionally wherein the pH of the granulation liquid is in the range of 5 to 7.

6. The process according to any one of the previous claims, wherein the acid is organic or inorganic acid.

7. The process according to claim 6, wherein the acid is selected from the group consisting of hydrochloric acid, citric acid, fumaric acid, maleic acid, malic acid, succinic acid, phosphoric acid and/or acetic acid.

8. The process according to claim 7, wherein the acid is hydrochloric acid and/or citric acid.

9. The process according to claim 8, wherein the acid is hydrochloric acid.

10. The process according to any one of the previous claims, wherein the granulation liquid in step b) is sprayed onto metformin hydrochloride only without additional intragranular excipients to prepare the granules.

11. The process according to any one of the previous claims, wherein the extragranular excipients are magnesium stearate and/or colloidal silicon dioxide.

12. The process according to any one of the previous claims, wherein the pH of the granulation liquid is in the range of 5 to 7, the acid is hydrochloric acid, the binder is copovidone and extragranular excipient is magnesium stearate.

13. The process according to any one of the previous claims, wherein the final dosage form is a tablet.

14. Solid pharmaceutical composition comprising linagliptin and metformin hydrochloride, which is obtainable by a process according to any one of the preceding claims 1 to 13.

15. Solid pharmaceutical composition comprising linagliptin and metformin hydrochloride, which is obtainable by a process according to any one of claims 1 to 13, for use in the treatment of diabetes mellitus type 2.

## Patentansprüche

1. Verfahren zur Herstellung einer festen pharmazeutischen Zusammensetzung umfassend Linagliptin und Metforminhydrochlorid, wobei das Verfahren die folgenden Schritte umfasst:
a) Herstellen einer Granulierungsflüssigkeit durch Dispergieren von Linagliptin, einer Säure und gegebenenfalls eines Bindemittels in Wasser;
b) Sprühen der Granulierungsflüssigkeit auf Metforminhydrochlorid gegebenenfalls gemischt mit intragranulare(m/n) Exzipient(en), wobei Granula hergestellt werden;
c) Trocknen der Granula;
d) gegebenenfalls Zugeben von extragranulare(m/n) Exzipient(en); und
e) Herstellen der finalen Dosierungsform.

2. Verfahren gemäß Anspruch 1, wobei die Granulierungsflüssigkeit auch ein Bindemittel umfasst.

3. Verfahren gemäß Anspruch 2, wobei das Bindemittel Bindemittel ausgewählt aus der Gruppe, welche aus Povidon, Copovidon, Hydroxypropylmethylcellulose, Hydroxypropylcellulose, Hydroxyethylcellulose, Methylcellulose, Polyvinylalkohol, Polyethylenglykol, Stärke und/oder Quellstärke besteht, ist oder umfasst.

4. Verfahren gemäß Anspruch 3, wobei das Bindemittel Copovidon ist.

5. Verfahren gemäß einem der vorhergehenden Ansprüche, wobei der pH-Wert der Granulierungsflüssigkeit im Bereich von 3 bis 8 liegt, gegebenenfalls wobei der pH-Wert der Granulierungsflüssigkeit im Bereich von 4 bis 7 liegt, ferner gegebenenfalls wobei der pH-Wert der Granulierungsflüssigkeit im Bereich von 5 bis 7 liegt.

6. Verfahren gemäß einem der vorhergehenden Ansprüche, wobei die Säure organische oder anorganische Säure ist.

7. Verfahren gemäß Anspruch 6, wobei die Säure aus der Gruppe, welche aus Chlorwasserstoffsäure, Zitronensäure, Fumarsäure, Maleinsäure, Äpfelsäure, Bernsteinsäure, Phosphorsäure und/oder Essigsäure besteht, ausgewählt ist.

8. Verfahren gemäß Anspruch 7, wobei die Säure Chlorwasserstoffsäure und/oder Zitronensäure ist.

9. Verfahren gemäß Anspruch 8, wobei die Säure Chlorwasserstoffsäure ist.

10. Verfahren gemäß einem der vorhergehenden Ansprüche, wobei die Granulierungsflüssigkeit in Schritt b) nur auf Metforminhydrochlorid ohne zusätzliche intragranulare Exzipienten gesprüht wird, um die Granula herzustellen.

11. Verfahren gemäß einem der vorhergehenden Ansprüche, wobei die extragranularen Exzipienten Magnesiumstearat und/oder kolloidales Siliciumdioxid sind.

12. Verfahren gemäß einem der vorhergehenden Ansprüche, wobei der pH-Wert der Granulierungsflüssigkeit im Bereich von 5 bis 7 liegt, die Säure Chlorwasserstoffsäure ist, das Bindemittel Copovidon ist und extragranularer Exzipient Magnesiumstearat ist.

13. Verfahren gemäß einem der vorhergehenden Ansprüche, wobei die finale Dosierungsform eine Tablette ist.

14. Feste pharmazeutische Zusammensetzung umfassend Linagliptin und Metforminhydrochlorid, welche durch ein Verfahren gemäß einem der vorhergehenden Ansprüche 1 bis 13 erhalten werden kann.

15. Feste pharmazeutische Zusammensetzung umfassend Linagliptin und Metforminhydrochlorid, welche durch ein Verfahren gemäß einem der Ansprüche 1 bis 13 erhalten werden kann, zur Verwendung in der Behandlung von Diabetes mellitus Typ 2.

## Revendications

1. Procédé de préparation d'une composition pharmaceutique solide comprenant de la linagliptine et du chlorhydrate de metformine, dans lequel le procédé comprend les étapes suivantes :
a) la préparation d'un liquide de granulation par dispersion de la linagliptine, d'un acide et éventuellement d'un liant dans de l'eau ;
b) la pulvérisation du liquide de granulation sur du chlorhydrate de metformine éventuellement mélangé avec un/des excipient(s) intragranulaire(s) pour préparer des granulés ;
c) le séchage des granulés ;
d) l'ajout éventuel d'excipient(s) extragranulaire(s) ; et
e) la préparation de la forme posologique finale.

2. Procédé selon la revendication 1, dans lequel le liquide de granulation comprend également un liant.

3. Procédé selon la revendication 2, dans lequel le liant est ou comprend un liant choisi dans le groupe constitué par la povidone, la copovidone, l'hydroxypropylméthylcellulose, l'hydroxypropylcellulose, l'hydroxyéthylcellulose, la méthylcellulose, l'alcool polyvinylique, le polyéthylèneglycol, l'amidon et/ou l'amidon prégélatinisé.

4. Procédé selon la revendication 3, dans lequel le liant est la copovidone.

5. Procédé selon l'une quelconque des revendications précédentes, dans lequel le pH du liquide de granulation est compris entre 3 et 8, éventuellement, dans lequel le pH du liquide de granulation est compris entre 4 et 7, éventuellement encore dans lequel le pH du liquide de granulation est compris entre 5 et 7.

6. Procédé selon l'une quelconque des revendications précédentes, dans lequel l'acide est un acide organique ou inorganique.

7. Procédé selon la revendication 6, dans lequel l'acide est choisi dans le groupe constitué par l'acide chlorhydrique, l'acide citrique, l'acide fumarique, l'acide maléique, l'acide malique, l'acide succinique, l'acide phosphorique et/ou l'acide acétique.

8. Procédé selon la revendication 7, dans lequel l'acide est l'acide chlorhydrique et/ou l'acide citrique.

9. Procédé selon la revendication 8, dans lequel l'acide est l'acide chlorhydrique.

10. Procédé selon l'une quelconque des revendications précédentes, dans lequel le liquide de granulation à l'étape b) est pulvérisé sur le chlorhydrate de metformine uniquement sans excipients intragranulaires supplémentaires pour préparer les granulés.

11. Procédé selon l'une quelconque des revendications précédentes, dans lequel les excipients extragranulaires sont le stéarate de magnésium et/ou le dioxyde de silicium colloïdal.

12. Procédé selon l'une quelconque des revendications précédentes, dans lequel le pH du liquide de granulation est compris entre 5 et 7, l'acide est l'acide chlorhydrique, le liant est la copovidone et l'excipient extragranulaire est le stéarate de magnésium.

13. Procédé selon l'une quelconque des revendications précédentes, dans lequel la forme posologique finale est un comprimé.

14. Composition pharmaceutique solide comprenant de la linagliptine et du chlorhydrate de metformine, qui peut être obtenue par un procédé selon l'une quelconque des revendications 1 à 13 précédentes.

15. Composition pharmaceutique solide comprenant de la linagliptine et du chlorhydrate de metformine, qui peut être obtenue par un procédé selon l'une quelconque des revendications 1 à 13, pour une utilisation dans le traitement du diabète sucré de type 2.
